# EUROPEAN PATENT APPLICATION

(11) **EP 3 399 313 A1**
(43) Date of publication of application: **07.11.2018**
(21) Application number: 17182293.5
(22) Date of filing: 20.07.2017
(51) Int. Cl.: G01N 33/50, B01L 3/00, G01N 21/75

(54) **DEVICE AND METHOD FOR MEASURING DIFFUSION OF A COMPOUND ACROSS A MEMBRANE**

(30) Priority: 03.05.2017 US 201762500741 P
(71) Applicant: Galderma Research & Development, 06410 Biot (FR)
(72) Inventor: ATRUX-TALLAU, Nicolas, 06600 ANTIBES (FR)
(74) Representative: Casalonga

(57) **Abstract**

Device (10) for measuring diffusion of a compound across a test membrane (40) comprises a donor assembly (20) comprising a donor plate (22) having at least one through hole (23) and a receptor assembly (30) comprising a receptor plate (32) having at least one receptor well (32a) configured for containing a receiving liquid, said receptor well mirroring the through hole (23), said donor plate (22) being located over the receptor plate (32) and configured to move along a vertical direction (Z) towards the receptor plate (32).

The donor assembly (20) comprises a donor chamber (24) mounted in said through hole (23) and extending on both side of the donor plate (22), said donor chamber (24) delimiting an inner cavity (24b) forming a donor well for receiving said compound to be tested and having a lower end (24c) configured to come into contact with said test membrane.

## Description

The present invention relates to the general field of in vitro testing for studying the penetration behaviour of products on a test membrane, such as skin.

More particularly, the present invention relates to a device and method for evaluating the penetration capacity in a test membrane of a compound, such as for example a bioactive agent, in a vehicle.

Typical systems for measuring diffusion of a compound in a test membrane comprises a first chamber and a second chamber separated from the first chamber by said test membrane. One of the chambers contains a compound to be tested, for example a pharmaceutical composition, in a vehicle, and the other chamber contains a receiving solution, such as for example physiological serum. The compound and the solution contact opposite surfaces of the test membrane. The concentration of the compound in the receiving solution is measured in order to determine the diffusion rate of the compound across the test membrane. Additionally the concentration of the compound within the test membrane can be determined through different technics.

However, such systems cannot perform multiple diffusion measurements in parallel using a single membrane.

Known devices for testing the diffusion of a compound within a vehicle through a test membrane generally comprise a receptor plate, a donor plate secured to said receptor plate and a test membrane captured between the receptor and donor plates. The donor plate comprises a plurality of through channels forming donor wells and the receptor plate comprises a plurality of non-through channels forming receiver wells which mirror the donor wells.

Referenced can be made to documents US 8, 277, 762 - A1 (Tioga Research) which describes such a device and method.

However in this document, in order to isolate the cells from one another, the test membrane is cut by ceramic blades when sandwiched between the receptor and donor plates. The test membrane is thus damaged.

The aim of the present invention is to overcome the aforementioned drawbacks.

One aim of the present invention is to provide a system and method for rapidly screening a large number of compounds or formulations while efficiently using a test membrane.

Another aim of the present invention is to obtain accurate membrane diffusion measurements by avoiding air gaps between the receiving solution and the test membrane in order.

The object of the present invention is to provide a device for measuring diffusion of a compound within and across a test membrane comprises a donor assembly comprising a donor plate having at least one through hole and a receptor assembly comprising a receptor plate having at least one receptor well configured for containing a receiving liquid, said receptor well mirroring the through hole and opens on an upper surface of said receptor plate. Said donor plate is located over the receptor plate and mobile in translation along a vertical direction compared to the receptor plate. In other words, the donor plate is configured to move towards the receptor plate along said vertical direction.

For example, all parts of the device are made in a material resisting to chemical product and able to be used in a steam pressure sterilization autoclave, such as for example stainless steel.

As an alternative, it is also possible to use plastic material, such as for example polytetrafluoroethylene (PTFE) for said device, which allows avoiding interaction between the device and the compound to be tested.

The donor assembly comprises a donor chamber mounted in said through hole and extending on both sides of the donor plate, said donor chamber delimiting an inner cavity forming a donor well for receiving said compound to be tested and having a lower end configured to come into contact with said test membrane.

The mobility of the donor chamber allows mating the upper surface of the test membrane.

For example, the inner diameter of the through-hole of the donor plate is bigger than the outer diameter of the donor chamber, so that the donor chamber can move freely along said vertical direction in said through-hole.

The donor assembly may further comprise a locking nut adapted to cooperate with a threaded end of the donor chamber, when screwed, said locking nut bearing on an upper surface of the donor plate. Thanks to said locking nut, the donor chamber cannot fall towards the receptor plate when the donor plate is lifted, and is thus retained vertically.

Each locking nut is for example provided on its periphery with a notch adapted to cooperate with a special tool to screw/unscrew the locking nut on the donor body.

In an embodiment, the donor assembly further comprises a pre-stressing element, for example a spring, located between the donor plate and the donor chamber and configured to exert an axial pre-stress (along the vertical direction) on the donor chamber when the donor plate is moved towards the receptor plate.

In an embodiment, a first end of said pre-stressing element is fixed to the donor plate and a second end of said pre-stressing element, opposite to said first end, is fixed to the locking nut, so that the force applied to the donor plate is transmitted to the donor chamber through the stretching of said pre-stressing element.

In a preferred embodiment, said pre-stressing element is located between an inner shoulder provided in the bore of the through-hole of the donor plate and an outer shoulder provided on the outer surface of the donor chamber. In other words, a first end of said pre-stressing element is fixed to the donor plate inside the through-hole and a second end of said pre-stressing element, opposite to said first end, is fixed to the donor chamber.

In an embodiment, the receptor plate comprises a transversal hole made along a transversal axis and coming into the receptor well, said transversal hole coming onto one lateral surface of the receptor plate.

In other words, the transversal hole provides access to a corresponding receptor well. Said hole is provided with a threaded part configured to cooperate with a threaded plug.

The transversal hole allows avoiding overpressure within the receptor well and thus avoiding blistering of the test membrane. When there is a plurality of receptor wells mirroring a plurality of donor wells, the receptor plate comprises a plurality of transversal holes made along a transversal axis and each coming into the corresponding receptor well, said transversal holes coming onto the two lateral surface of the receptor plate. It is thus possible to open or close each receptor well individually.

It is also possible to connect each hole with a tubing having a screw pipe, for example, in view of collecting the liquid contained in each receptor well after diffusion of the compound.

In another embodiment, the receptor plate comprises a longitudinal channel made along a longitudinal axis and having two ends. At least one end of said longitudinal channel opens on a lower surface, opposite to the upper surface, of the receptor plate, said channel being fluidly connected to the receptor well.

When there is a plurality of receptor wells mirroring a plurality of donor wells, the channel is fluidly connected to all the receptor wells.

For example, both ends of said channel open onto the lower surface of the receptor plate.

Said only one channel is configured to receive an under pressure fluid from a pipe connected to one or both ends of said channel opening on the lower surface of the receptor plate, so that when an under pressure fluid is inserted in each end of the channel, the fluid pushes the liquid contained in each receptor well after diffusion of the compound towards the transversal holes. Thanks to the under pressure fluid there is no possible flow of liquid between each receptor well.

For example, said channel may also open onto one or both of two transversal faces of the receptor plate.

For example, said channel has a threaded part designed to cooperate with a threaded lid, for closing said channel.

The receptor assembly may comprise at least three legs, for example four.

In an embodiment, the device comprises at least two tightening systems each comprising a vertical screw pivotably attached to one side of the receptor plate, for example with a pin secured to said receptor plate, and a tightening nut bearing on the outer surface of the donor plate, via a bushing or directly.

When screwing/unscrewing each tightening systems, the donor plate is pushed towards the receptor plate and provides a force to the said pre-stressing element thus allowing exerting individual smooth pressure onto the test membrane.

Indeed, the outer surface of the test membrane or the test membrane itself may not be totally flat; such pressure compensation system allows constant pressure repartition through the test membrane and avoids the test membrane to be damaged.

Each of said tightening systems is, for example, located at each lateral side of the device, the access to the longitudinal channel being thus difficult through the transversal faces of the receptor plate. Thanks to the opening of said longitudinal channel onto a lower surface of the receptor plate, access is easier for plugging a pipe transmitting an under pressure fluid into said channel.

Said device may also comprise at least one sliding guide projecting from the receptor plate into a hole provided in the donor plate so that the donor plate may slide on said sliding guide along the vertical axis.

As a non-limitative example, the number of sliding guides can be two or four. For example, bushings surrounding the sliding guide may be provided to make the sliding of the donor plate more efficient.

For example, the donor chamber has an elongated body having a lower end configured to come in contact with an upper surface of the test membrane, an upper end, opposite to said lower end and having at least one outer thread, and an intermediate part located between said lower end and said upper end and having an outer diameter bigger than the outer diameter of the upper end.

The outer diameter of the intermediate part is smaller than the inner diameter of the through-hole of the donor plate.

Said threaded upper end is configured to cooperate with the locking nut.

For example, said threaded upper end is also configured to cooperate with a locking cap. The locking cap allows to work under occlusive conditions. It is thus possible to close independently all the donor chambers when there are a plurality of said donor chambers.

The locking cap has, for example, on its periphery a slot adapted to cooperate with a special tool to screw/unscrew the cap on the donor body.

As a non-limitative example, the donor chamber has a shoulder located between the intermediate part and the lower end and having an outer diameter bigger than the outer diameter of the lower end, and thus bigger than the inner diameter of the through-hole of the donor plate.

In an embodiment, the donor chamber comprises, at its lower end, an O-ring.

For example, said O-ring is located in a circular notch provided on the lower end of the donor chamber.

Thanks to such O-ring, leakage between each donor wells in contact with the membrane can be prevented. Each donor wells are thus tightly sealed onto the membrane.

The O-ring may be for example in silicon and may have 2mm thickness.

Each donor chamber may comprise a bushing resting on the shoulder of the donor chamber, the lower end of the pre-stressing element being located between the upper surface of said bushing and the lower surface of the donor plate.

The outer surface of the intermediate part may, for example, be provided with a flat section. As an alternative, the outer surface of the whole elongated body may be cylindrical.

In an embodiment, the donor plate comprises a plurality of through-out holes made in the thickness of the donor plate along the vertical direction, each receiving one donor chamber and the receptor plate comprises a plurality of receptor wells, each mirroring a corresponding donor well.

It is thus possible, using a single test membrane, to have a plurality of penetration measurements, without damaging said test membrane.

For example, each through-out hole has a cylindrical bore.

The test membrane may be synthetic polymer, animal or human tissue or skin or in vitro reconstructed tissue. The test membrane could also be isolated epidermis, full thickness skin without epidermis, etc...

According to a second aspect, the present invention relates to a method for measuring diffusion of a compound within or across a test membrane using the device as described above. The method comprises a step of positioning a test membrane on the upper surface of the receptor plate, a step of sliding the donor plate towards the receptor plate, for example by screwing tightening systems, until the donor chambers contact the outer surface of the test membrane, a step of inserting a receiving solution within each receptor well, for example using the transversal holes. As an alternative, the receiving solution may be put in each receptor well before positioning the test membrane on the receptor plate.

The method further comprises a step of inserting a compound within each donor wells. The compounds to be tested may be different from one donor wells to the other. It is thus possible to have a large number of compounds to be tested. It is also possible to test the penetration of one compound within a plurality of vehicles.

The method further comprises a step of incubating the device during a predetermined period, for example, it is possible to apply a temperature comprised between 32°C and 37°C, for example equal to 35°C during said period, a step of removing excess of compound on the surface of the membrane, for example by washing, a step of separating the donor assembly from the receptor plate, a step of striping the test membrane, for example using adhesive material to ensure excess removal, a step of collecting a biopsy punch at each exposed area, and a step of analysing said biopsy punches.

In an embodiment, during or following the incubation period, liquid contained in each receptor well after diffusion of the compound is collected, for example via the through holes made along the transversal direction.

The present invention will be better understood from studying the detailed description of an embodiment considered by way of a nonlimiting example and illustrated by the attached drawings in which:
- Figure 1 is a perspective view of a device according to an embodiment of the present invention;
- Figure 2 is a top view of the device of Figure 1;
- Figure 3 is a cross-section on III-III of Figure 2;
- Figure 4 is a cross-section on IV-IV of Figure 2;
- Figure 5 is a side view of the device of Figure 1;
- Figure 6 is a cross-section on VI-VI of Figure 5;
- Figure 7 is a cross-section on VII-VII of Figure 6;
- Figure 8 is a perspective view of a donor chamber of the device of Figure 1 according to one embodiment;
- Figure 9 is a cross section a donor chamber of the device of Figure 1 according to another embodiment;
- Figure 10 is a cross-section on IV-IV of Figure 2 according to another embodiment; and
- Figure 11 is a flow chart of a method for measuring diffusion of one compound or a plurality of compounds across a test membrane using the device of Figures 1 to 9.

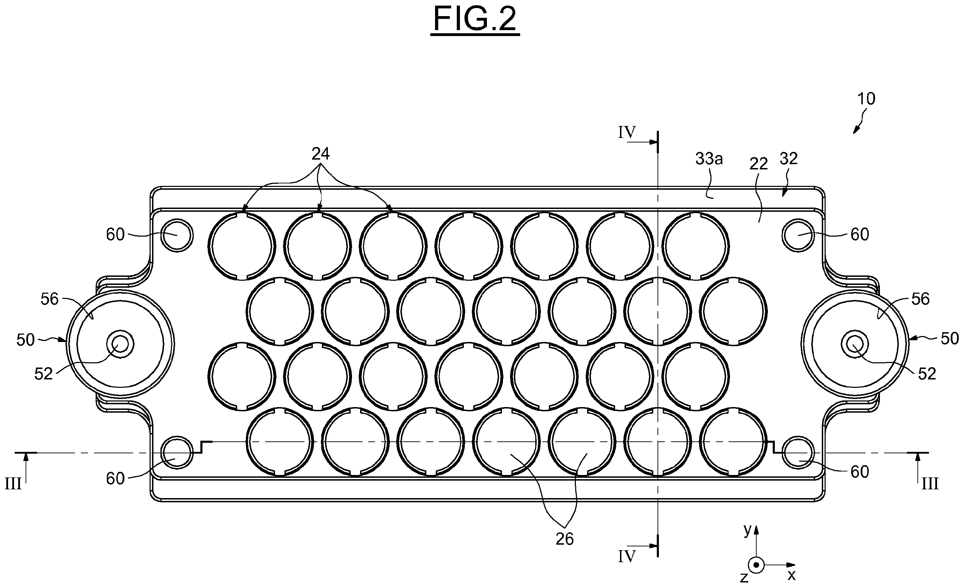

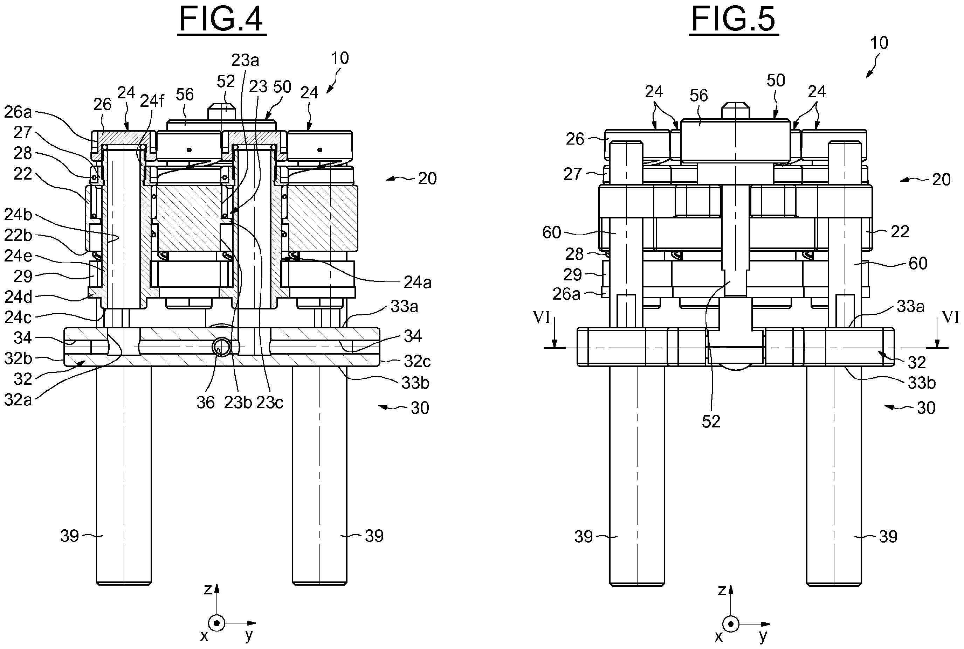

In the further description, terms "longitudinal", "transversal", "vertical", "front", "back", "left", and "right" are defined according to the usual orthogonal bench mark, illustrated on the Figures, and including:
- a longitudinal axis X, horizontal and directed from the left to the right of Figure 2;
- a transversal axis Y, horizontal, perpendicular to the longitudinal axis X and directed from the left to the right of Figure 5 ;
- a vertical axis Z, orthogonal with the longitudinal and the transversal axis X,Y.

As illustrated on the Figures, a device for measuring diffusion of one compound or a plurality of compounds within and or across a test membrane is designed by general reference number 10.

Said device 10 is either a static device for determination of membrane penetration either a dynamic flow through device for determination of penetration and permeation of compounds using biological membrane in vitro.

The device 10 extends along the longitudinal axis X and comprises a donor assembly 20 and a receptor assembly 30. A test membrane 40, made for example of synthetic material, animal tissue or skin, is located between the donor and receptor assemblies 20, 30. The donor assembly 20 is located over the receptor assembly 30 and is configured to move along the vertical direction Z towards the receptor assembly.

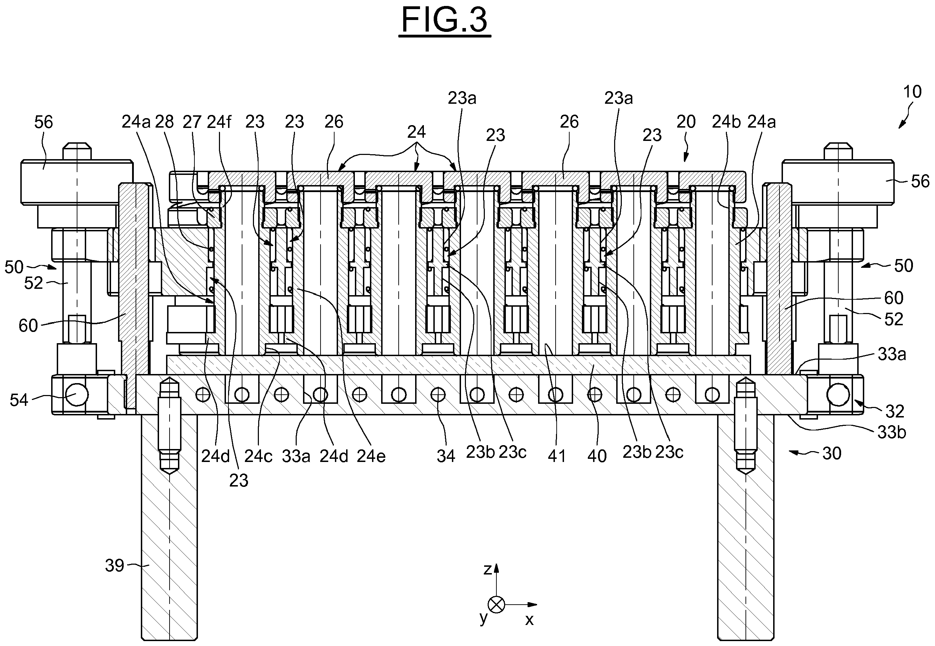

For sake of clarity, said test membrane 40 is only shown on Figure 3.

The donor assembly 20 comprises a donor plate 22 provided with a plurality of through-out holes 23 made in the thickness of the donor plate 22 along the vertical direction Z and a plurality of donor chambers 24 each mounted in one through-hole 23. As illustrated, each through-out hole 23 have a cylindrical stepped bore.

As can be seen on Figures 3 and 4, each through-hole 23 comprises a first bore 23a, a second bore 23b connected to said first bore 23a by an inner shoulder 23c. Said shoulder 23c has an inner diameter smaller than the inner diameter of the first and second bores 23a, 23b.

However, it is possible to provide through-holes having a uniform cylindrical bore.

It is also possible to have other shapes for the through-holes, as for example of rectangular cross section. Each through-out hole 23 is configured to receive one donor chamber 24.

Each independent donor chamber 24 have an elongated body 24a extending on both side of the donor plate 22 and delimiting an inner cavity 24b forming a donor well for receiving a compound within a vehicle to be tested. As illustrated, the cavity 24b has a substantially cylindrical shape, but could have any other shape. As illustrated, the donor plate 22 has twenty height through-holes 23. As an alternative, the number of though-holes and donor chambers may be different, for example comprised between one and twenty-seven or higher than twenty eight.

The elongated body 24a has a lower end or part 24c configured to come in contact with an outer surface 41 of the test membrane, a shoulder 24d having an outer diameter bigger than the outer diameter of the lower end 24c, an intermediate part 24e having an outer diameter smaller than the outer diameter of the shoulder 24d but bigger than the outer diameter of the lower end 24c, and an upper end 24f, opposite to said lower end 24c and having an outer diameter smaller than the outer diameter of the intermediate part 24e. The upper end 24f is threaded in order to cooperate with inner threads of a locking cap 26. The locking cap 26 has on its periphery a slot 26a adapted to cooperate with a special tool (not shown) to screw/unscrew the cap on the donor body. Said cap allows to work under occlusive conditions. As an alternative, the elongated body may not be provided with said shoulder 24d.

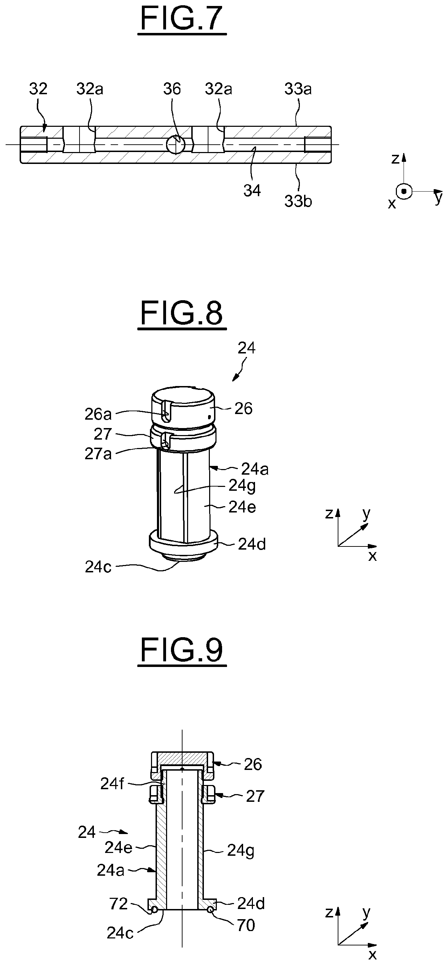

As can be seen on Figure 8, the outer surface of the elongated body, notably of the intermediate part 24e, is provided with a flat section 24g. As an alternative, the outer surface of the elongated body in its whole may be cylindrical.

As illustrated, the inner diameter of the through-holes 23 of the donor plate 22, i.e. the bores 23a, 23b and the shoulder 23c, is slightly bigger than the outer diameter of the elongated body 24a of the donor chamber 24, so that each donor chamber can move freely along the vertical direction in the corresponding through-hole 23. When the elongated body is provided with an outer shoulder 24d, the outer diameter of said outer shoulder is slightly bigger than the inner diameter of the through-hole 23, notably of the second bore 23b.

Each donor chamber 24 further comprises a locking nut 27 adapted to cooperate with the threaded end 24f of the elongated body 24a. A pre-stressing element 28, such as for example a spring, is located between the donor plate 22 and the donor chamber 24 and configured to exert an axial pre-stress (along the vertical direction) on the donor chamber 24 when the donor plate 22 is moved towards the receptor plate.

As can be seen on Figure 4, said pre-stressing element 28 is located between a lower surface 22b of the donor plate 22 and the upper surface of the locking nut 27. In other words, a first end of said pre-stressing element 28 is fixed to the donor plate 22 while a second end of said pre-stressing element 28, opposite to said first end, is fixed to the locking nut 27, so that the force applied to the donor plate is transmitted to the donor chamber through the stretching of said pre-stressing element.

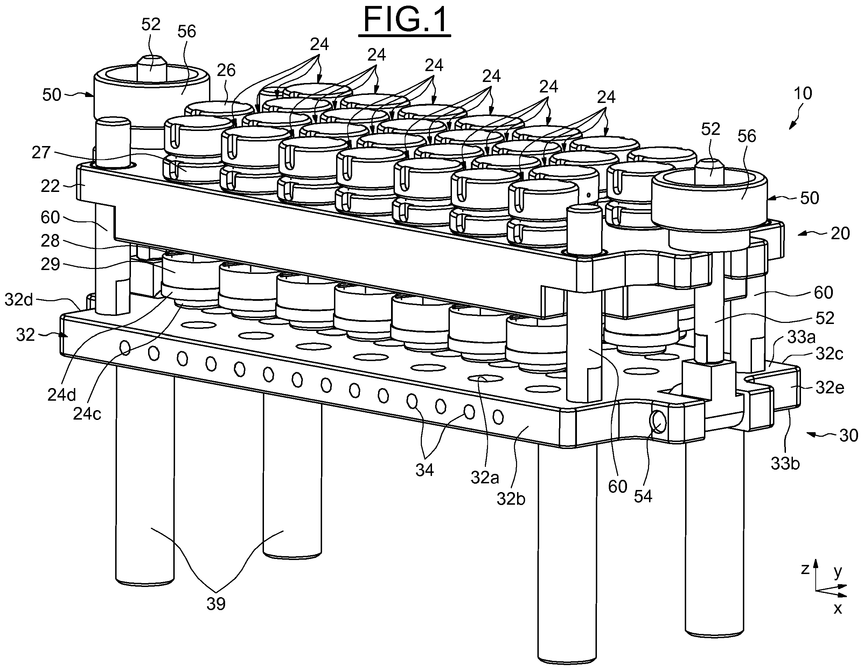

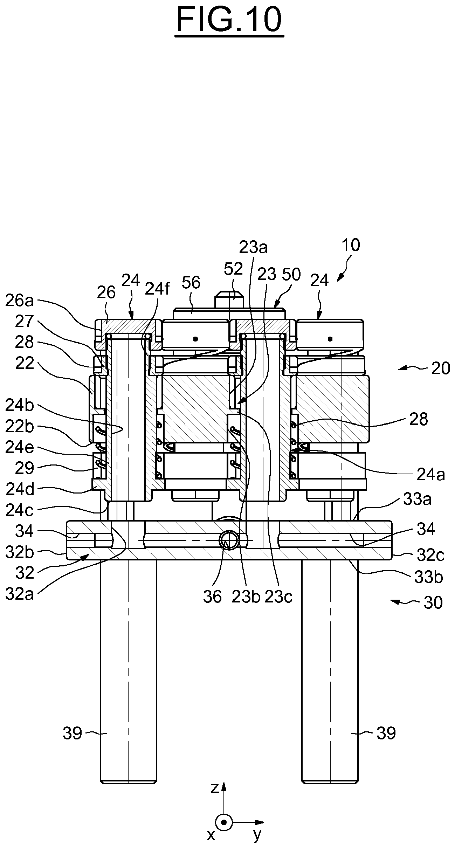

In another embodiment, shown on Figure 10, in which the same elements bear the same reference, differs from the embodiment shown on Figures 1 to 9, only on the location of the pre-stressing element 28. Said pre-stressing element 28 is located between the lower surface of the inner shoulder 23c provided in the stepped bore of the through-hole 23 of the donor plate 22 and the upper surface of the outer shoulder 24d provided on the outer surface of the donor chamber 24.

In both cases, the donor chambers 24 are thus pre-stressed along the vertical axis Z when the donor plate 22 is moved towards the receptor plate 32.

Each locking nut 27 is further provided on its periphery with a notch 27a adapted to cooperate with a special tool (not shown) to screw/unscrew the locking nut 27 on the donor body. When screwed, the locking nut 27 bears vertically on the donor plate 22.

As a non-limitative example, each donor chamber 24 further comprises a bushing 29 resting on the shoulder 24d of the body 24, the lower end of the spring 28 being located between the upper surface of said bushing 29 and the lower surface 22b of the donor plate 22.

The receptor assembly 30 comprises a receptor plate 32. The donor plate 22 is mobile in translation along the vertical direction Z compared to said receptor plate 32. In other words, the donor plate 22 is configured to move towards the receptor plate 32 along said vertical direction Z.

The receptor plate 32 has a plurality of non through-out holes 32a forming receptor wells made in the thickness of the receptor plate 32 along the vertical direction Z and opening on the upper surface 33a of the receptor plate 32. As illustrated, each receptor wells 32a have a cylindrical bore. However, it is possible to have other shapes for the receptor wells 32a, as for example of rectangular cross section. Each receptor well 32a is filled with a receiving solution, such as, for example physiological serum. The receptor wells 32a mirror or face the donor wells 24b of the donor chambers 24. The test membrane 41 is positioned on the upper surface 33a of the receptor plate 32. The compound and the receiving solution thus contact opposite surfaces of the test membrane 41.

For example, each receptor well 32a have an inner bore of 6mm diameter and 5mm depth.

The receptor plate 32 further comprises on its lateral surfaces 32b, 32c, a plurality of transversal holes 34 made along the transversal axis Y, each coming into a corresponding receptor well 34. In other words, each transversal hole 34 provides access to a corresponding receptor well 32a. Said transversal holes 34 are provided with a threaded part (not referenced), as shown on Figure 7, configured to cooperate with a threaded plug (not shown).

The transversal holes 34 allow avoiding overpressure within the receptor wells 32a and thus avoiding blistering of the test membrane. It is thus possible to open or close each receptor well 32a individually.

It is also possible to connect each hole 34 with a tubing having a screw pipe, for example, in view of collecting the liquid contained in each receptor well 32a after diffusion of the compound.

The receptor plate 32 further comprises a longitudinal channel 36 made along the longitudinal axis X and fluidly connected to each receptor well 32a. As an alternative, only one end of the channel 36 may open onto the lower surface of the receptor plate.

Said single channel 36 is configured to receive an under pressure fluid from a pipe (not shown) connected to one or both ends of said channel 36 opening on the lower surface 33b of the receptor plate 32. When an under pressure fluid is inserted in one or both ends of the channel 36, the fluid pushes the liquid contained in each receptor well 32a after diffusion of the compound towards the transversal holes 34. Thanks to the under pressure fluid there is no possible flow of liquid between each receptor well 32a.

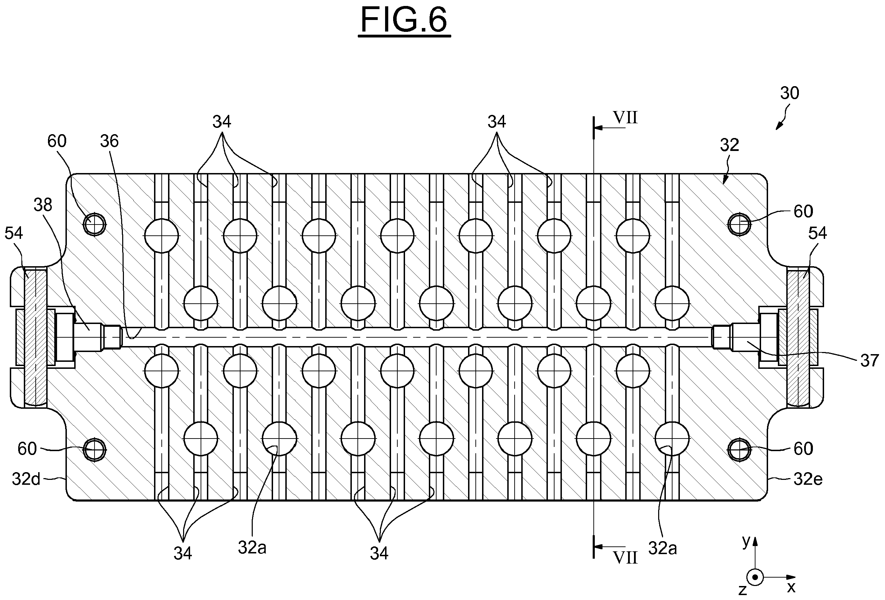

As can be seen on Figure 6, said channel 36 may also open onto one or both of two transversal faces 32d, 32e of the receptor plate 32.

As illustrated, said channel has a threaded part (not referenced) designed to cooperate with a threaded lid 37 shown on figure 6, for closing said ends of the channel 36 opening onto the transversal faces 32d, 32e.

As a non-limitative way, the receptor assembly comprises four legs 39.

The device 10 further comprises two tightening systems 50 each comprising a vertical screw 52 pivotably attached to one side of the receptor plate 32 with a pin 54 secured to said plate 32. Each tightening systems 50 further comprises a tightening nut 56 bearing on the outer surface of the donor plate 22, via a bushing or directly.

When screwing/unscrewing said tightening systems 50, the donor plate 22 is pushed towards the receptor plate 32 and provides a force to the said spring 28 thus allowing exerting individual smooth pressure onto the test membrane 40.

Indeed, the outer surface of the test membrane or the test membrane itself may not be totally flat; such pressure compensation system allows constant pressure repartition through the test membrane and avoids the test membrane to be damaged.

Each of said tightening systems 50, notably its pin 54, is located at each lateral side of the device 10 rendering the access to the longitudinal channel 36 particularly difficult through the transversal faces 32d, 32e of the receptor plate 32. Thanks to the opening of said longitudinal channel onto the lower surface 33b of the receptor plate 32, access is easier for plugging a pipe (not shown) to said openings and transmitting an under pressure fluid into said channel 36.

As illustrated, the device 10 further comprises sliding guides 60 projecting from the receptor plate 32 into a hole provided in the donor plate 22 so that the donor plate 22 may slide on said sliding guides. As a non-limitative example, the number of sliding guides is four. However, the number of sliding guides could be for example equal to two. Bushings (not shown) could also be provided to make the sliding more efficient.

In the embodiment shown in Figure 9 in which the same element are designed by the same reference, the donor chamber 24 comprises, at its lower end 24c, a O-ring 70 located in a circular notch 72 provided on the lower end of the shoulder 24d, or directly on the lower end of the elongated body. Thanks to such O-ring, leakage between each donor wells in contact with the membrane can be prevented. Each donor wells are thus tightly sealed onto the membrane. The O-ring 70, may be for example in silicon and may have 2mm thickness.

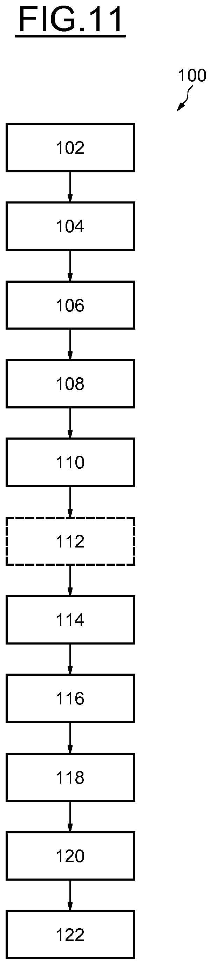

The flow chart illustrated on Figure 11 shows the different steps of a method 100 for measuring diffusion of one compound or a plurality of compounds across a test membrane using the device of Figures 1 to 10.

In a first step 102, a test membrane 40 is positioned on the upper surface of the receptor plate 32. For example, the test membrane can be defatted when it is animal tissue or human skin.

By way of screwing the tightening systems 50 at step 104, the donor assembly 20 is slided towards the receptor plate until the donor chambers 24 contact the outer surface of the test membrane 40.

A receiving solution is put, at step 106, within each receptor well 32a, using the transversal holes 34. As an alternative, the receiving solution may be put in each receptor well 32a before positioning the test membrane on the receptor plate 32, in order to avoid formation of air bubbles between the test membrane and the receiving solution.

At step 108, a compound within a vehicle is inserted in each donor wells 24b. The compounds to be tested may be different from one donor wells to the other. It is thus possible to have a large number of compounds to be tested. It is also possible to test the penetration of one compound within a plurality of vehicles.

At step 110, the device 10 is incubated during a predetermined period. For example, it is possible to apply a temperature of 35°C during said period.

Following incubation, liquid contained in each receptor well 32a after diffusion of the compound can be collected at step 112. It is also possible to collect said liquid during the incubation period, or not to collect said liquid.

At step 114, excess of compound on the surface of the membrane is removed, for example by washing and the donor assembly 20 is separated from the receptor plate 32 at step 116.

The test membrane is then stripped at step 118, for example using adhesive material to ensure excess removal and a biopsy punch is then collected at step 120 and extracted with a solvent for dosage at step 122 or cut using a microtome for obtaining slices quantified by adequate quantitative imagery technics for distribution assessment.

Thanks to the present invention, it is possible to perform multiple diffusion measurements in parallel using a single membrane, without damaging said membrane.

It is also possible to collect the liquid contained in each receptor well 32a independently after diffusion of the compound through the test membrane. It is also possible to assess compound permeation through the test membrane during experiments using flow through under pressure of receiving solution and kinetic collection of samples.

Finally, thanks to the device according to the invention, each donor well is tightly sealed to the test membrane by mating the outer surface of said membrane.

## Claims

1. Device (10) for measuring diffusion of a compound across a test membrane (40) comprises a donor assembly (20) comprising a donor plate (22) having at least one through hole (23) and a receptor assembly (30) comprising a receptor plate (32) having at least one receptor well (32a) configured for containing a receiving liquid, said receptor well mirroring the through hole (23), said donor plate (22) being located over the receptor plate (32) and configured to move along a vertical direction (Z) towards the receptor plate (32),
**characterized in that** the donor assembly (20) comprises a donor chamber (24) mounted in said through hole (23) and extending on both side of the donor plate (22), said donor chamber (24) delimiting an inner cavity (24b) forming a donor well for receiving said compound to be tested and having a lower end (24c) configured to come into contact with said test membrane.

2. Device (10) according to claim 1, wherein the inner diameter of the through-hole (23) of the donor plate (22) is bigger than the outer diameter of the donor chamber (24), so that the donor chamber can move freely along the vertical direction (Z) in said through-hole (23).

3. Device (10) according to claim 1 or 2, wherein the donor assembly (20) further comprises a locking nut (27) adapted to cooperate with a threaded end (24f) of the donor chamber (24), when screwed, said locking nut (27) bearing on an upper surface of the donor plate (22).

4. Device (10) according to any of the preceding claims, wherein the donor assembly (20) further comprises a pre-stressing element (28) located between the donor plate (22) and the donor chamber (24), said pre-stressing element exerting an axial pre-stress on the donor chamber (24) when the donor plate (22) is moved towards the receptor plate (32).

5. Device (10) according to claims 3 and 4, wherein a first end of said pre-stressing element (28) is fixed to the donor plate (22) and a second end of said pre-stressing element (28), opposite to said first end, is fixed to the locking nut (27).

6. Device (10) according to claim 4, wherein said pre-stressing element (28) is located between an inner shoulder (23c) provided in the bore of the through-hole (23) of the donor plate (22) and an outer shoulder (24d) provided on the outer surface of the donor chamber (24).

7. Device (10) according to any of claims 4 to 6, wherein said pre-stressing element (28) is a spring.

8. Device (10) according to any of the preceding claims, wherein the receptor plate (32) comprises a transversal hole (34) made along a transversal axis (Y) and coming into the receptor well (32a), said transversal hole (34) coming onto one lateral surface (32b, 32c) of the receptor plate (32).

9. Device (10) according to any of the preceding claims, wherein the receptor plate (32) comprises a longitudinal channel (36) made along a longitudinal axis (X), at least one end of said longitudinal channel (36) opens on a lower surface (33b) of the receptor plate (32), said channel (36) being fluidly connected to the receptor well (32a).

10. Device (10) according to claim 9, wherein both ends of said channel (36) open onto said lower surface (33b) of the receptor plate (32).

11. Device (10) according to any of the preceding claims, comprising at least two tightening systems (50) each comprising a vertical screw (52) pivotably attached to one side of the receptor plate (32), and a tightening nut (56) bearing on the outer surface of the donor plate (22).

12. Device (10) according to any of the preceding claims, comprising at least one sliding guide (60) projecting from the receptor plate (32) into a hole provided in the donor plate (22) so that the donor plate (22) may slide on said sliding guide.

13. Device (10) according to any of the preceding claims, wherein the donor chamber (24) has an elongated body (24a) having a lower end (24c) configured to come in contact with an outer surface (41) of the test membrane (40), an upper end (24f), opposite to said lower end (24c) and having at least one outer thread, and an intermediate part (24e) located between said lower end (24c) and said upper end (24f) and having an outer diameter bigger than the outer diameter of the upper end (24f).

14. Device (10) according to any of the preceding claims, wherein the donor chamber (24) comprises, at its lower end (24c), an O-ring (70).

15. Device (10) according to any one of the preceding claims, wherein the donor plate (22) comprises a plurality of through-out holes (23) made in the thickness of the donor plate (22) along the vertical direction (Z), each receiving one donor chamber (24) and the receptor plate (32) comprises a plurality of receptor wells (32a), each mirroring a corresponding donor well (24b).

16. Method (100) for measuring diffusion of a compound across a test membrane using the device (10) of claims 1 to 15, comprising the following steps:
- positioning a test membrane (40) on the upper surface of the receptor plate (32),
- sliding the donor plate (22) towards the receptor plate (32), until the donor chambers (24) contact the outer surface of the test membrane (40),
- inserting a receiving solution within each receptor well (32a),
- inserting a compound within each donor wells (24b),
- incubating the device (10) during a predetermined period.
- removing excess of compound on the surface of the membrane,
- separating the donor assembly (20) from the receptor plate (32).
- striping the test membrane
- collecting a biopsy punch, and
- analysing said biopsy punch.

17. Method according to claim 16, wherein, during or following the incubation period, liquid contained in each receptor well (32a) after or during diffusion of the compound is collected.
